# EUROPEAN PATENT APPLICATION

(11) **EP 1 306 883 A1**
(43) Date of publication of application: **02.05.2003**
(21) Application number: 01947851.0
(22) Date of filing: 05.07.2001
(51) Int. Cl.: H01J 61/35

(54) **TREATING APPARATUS UTILIZING ULTRAVIOLET RAY**

(30) Priority: 07.07.2000 JP 2000207237
(71) Applicant: Photoscience Japan Corporation, Hachioji-shi, Tokyo 193-0832 (JP)
(72) Inventor: NAKANO, Koji, c/o PHOTOSCIENCE JAPAN CORPORATION, Hachioji-shi, Tokyo 193-0832 (JP)
(74) Representative: Kehl, Günther, Dipl.-Phys.
(86) International application number: JP0105845
(87) International publication number: WO02005311

(57) **Abstract**

There is provided a light diffusing structure that diffuses ultraviolet rays, emitted by an ultraviolet ray discharge lamp (50), to irradiate a to-be-treated substance with the thus-diffused ultraviolet rays, so as to lessen unevenness in luminance of the rays. The light diffusing structure is provided by forming a generally while-colored thin film (11) on the inner surface of a light emitting tube (10) or minute irregularities on the inner or outer surface of the light emitting tube (10), or by forming a light-transmissive outer tube (protective tube) (60), accommodating therein the discharge lamp (50), of a translucent (diffusion-permeable) material. Such arrangements achieve enhanced sterilizing/purifying capabilities commensurate with amounts of ultraviolet ray irradiation by a high-density ultraviolet ray light source and can thereby perform efficient treatment.

## Description

### Technical Field

The present invention relates to an apparatus for performing purification, such as sterilization or disinfection, of water, air or the like using ultraviolet ray energy.

### Background Art

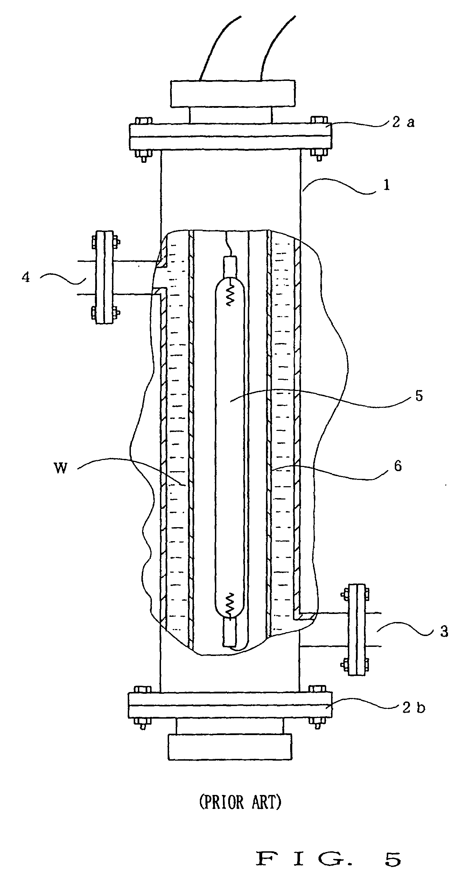

Ultraviolet rays of a short wavelength range are popularly used today for a variety of purposes, such as sterilization and decomposition of toxic and organic substances. Fig. 5 shows an example of a conventional liquid sterilization apparatus using ultraviolet rays, in which a cylinder-shaped tank 1 made of stainless steel has upper and lower ends closed with flanges 2a and 2b in a liquid-tight manner. The tank 1 also has a water inlet port 3 in its lower end portion and a water outlet port 4 in its upper end portion. Water W to be treated by the sterilization apparatus, introduced into the tank 1 through the water inlet port 3, is caused to flow upward in the tank 1 and then discharged through the water outlet port 4. Reference numeral 5 represents an ultraviolet ray light source, which is a low-pressure mercury vapor discharge lamp, medium-pressure mercury vapor discharge lamp or high-pressure mercury vapor discharge lamp that is generally capable of efficiently irradiating sterilizing ultraviolet rays of a 254 nm wavelength. The ultraviolet ray light source 5, i.e. discharge lamp 5, is inserted in an outer tube or protective tube 6 and isolated or insulated so as not to directly contact the to-be-treated water in the tank 1. In general, the outer tube or protective tube 6 is made of quartz glass highly permeable to ultraviolet rays. Upon powering-on of the discharge lamp 5 via a predetermined ballast (not shown), the discharge lamp 5 is illuminated to emit ultraviolet rays peculiar to mercury. The thus-emitted ultraviolet rays pass through the outer tube 6 and are irradiated to the to-be-treated water in the tank 1. The irradiated ultraviolet rays act on the DNA (deoxyribonucleic acid) of bacteria to exert sterilizing effects.

In recent years, efforts have been made to increase the density of the ultraviolet ray light source in order to achieve enhanced processing capabilities. For example, the above-mentioned medium-pressure mercury vapor discharge lamp is being used more frequently as a compact and high-density light source, because it can be constructed as a large-capacity discharge lamp although its sterilizing-ray irradiation efficiency is slightly lower that that of the low-pressure mercury vapor discharge lamp.

However, in the case where such a high-density light source is employed, there would arise the inconvenience that the sterilizing and other processing capabilities are not necessarily enhanced in proportion to the increased ultraviolet ray irradiation amounts.

### Disclosure of the Invention

In view of the foregoing, the present invention seeks to provide an efficient treating apparatus which achieves enhanced sterilizing and other processing capabilities commensurate with ultraviolet ray irradiation amounts.

The treating apparatus utilizing ultraviolet rays, provided by the present invention, is characterized by including a light diffusing structure that diffuses ultraviolet rays, emitted by an ultraviolet ray light source, to irradiate a to-be-treated substance with the diffused ultraviolet rays, so as to lessen unevenness in the luminance of the rays. Such arrangements allow the ultraviolet rays, emitted by the ultraviolet ray light source, to be effectively diffused by the light diffusing structure so that a to-be-treated substance is irradiated with the thus-diffused ultraviolet rays, and thus the present invention can lessen unevenness in the luminance of the ultraviolet rays to be irradiated to the to-be-treated substance. Even where a high-density light source is used, unevenness in the luminance of ultraviolet rays can be effectively lessened, so that the to-be-treated substance can be irradiated with ultraviolet rays with minimum luminance unevenness. As a result, the present invention can perform the purifying processing with improved purifying effects, solving the prior art problem that the sterilizing capabilities can not necessarily be enhanced in proportion to ultraviolet ray irradiation amounts.

Namely, as a result of analysis of the prior art problem, the inventor of the instant application has come to the conclusion that the reason why the processing capabilities, such as a sterilizing capability, are not necessarily enhanced in proportion to an increase in ultraviolet irradiation amounts may be unevenness in the luminance of ultraviolet rays irradiated to a to-be-treated substance. Namely, in general, a compact high-density light source tends to cause luminance unevenness due to increased discharge currents, so that the ultraviolet rays are not irradiated uniformly or evenly to the to-be-treated substance (such as a liquid, gas or solid) and processing capabilities, such as a sterilizing capability, are not necessarily enhanced in proportion to an increase in ultraviolet ray irradiation amounts. Such a tendency becomes particularly conspicuous when a uniformly-diffused substance, such as a liquid or gas, is to be treated. Namely, because a greater portion of the increased ultraviolet ray irradiation tends to be applied to only a part of the to-be-treated substance, the remaining part of the to-be-treated substance would not sufficiently receive the benefit of the increased ultraviolet ray irradiation amounts. Thus, the present invention is equipped with the light diffusing structure that is constructed to diffuse ultraviolet rays, emitted by the ultraviolet ray light source, to irradiate a to-be-treated substance with the diffused ultraviolet rays, so as to lessen unevenness in the luminance of the rays irradiated to the to-be-treated substance. With such a light diffusing structure, as great a part of the to-be-treated substance as possible can be irradiated with the ultraviolet rays uniformly, and the invention can thereby greatly enhance the processing capabilities for sterilization or the like of the to-be-treated substance. Particularly, if a high-density light source is employed, the present invention allows a much greater part of the to-be-treated substance to sufficiently receive the benefit of increased ultraviolet ray irradiation amounts, and can thereby have enhanced processing capabilities commensurate with the increased ultraviolet ray irradiation amounts.

As an embodiment, the light diffusing structure is provided on the inner or outer surface of a light emitting tube of the ultraviolet ray light source itself. For example, the light diffusing structure may be provided by forming a generally while-colored thin film on the inner surface of the light emitting tube (e.g., quartz-glass tube) of the ultraviolet ray light source, or minute irregularities or depressions and projections on the inner or outer surface of the light emitting tube. In another alternative, the light diffusing structure may be provided by forming the light emitting tube of a translucent (diffusion-permeable) material, such as a polycrystal alumina ceramic material. The light diffusing structure may be in any other suitable form than the above-mentioned. With such an arrangement, the present invention can form a diffusing surface that is capable of minimizing a loss of the ultraviolet rays; thus, even where the apparatus is used with no outer tube (protective tube) (as in a case where a gas is to be sterilized), the present invention can be applied effectively. Further, the white-colored thin film formed on the inner surface of the discharge lamp is preferable in that it will never peel off even when a hand of a human operator or some other object hits the outside of the discharge lamp during handling of the lamp.

The generally while-colored thin film formed on the inner surface of the glass tube may have, as its main constituent, at least one metal oxide selected from among a group consisting of an aluminum oxide, silicon oxide, calcium oxide, magnesium oxide, yttrium oxide, zirconium oxide and hafnium oxide. Using such a main constituent can form a while-colored thin film that is strong and chemically stable.

As another embodiment, the light diffusing structure may be provided by accommodating the ultraviolet ray light source in a light-transmissive protective tube and forming minute irregularities on the inner or outer surface of the protective tube. In an alternative, the light diffusing structure may be provided by forming the protective tube of a translucent ceramic material (such as a polycrystal alumina ceramic material). Thus, the same protective tube can continue to be used even when a discharge lamp, functioning as the ultraviolet ray light source, is to be replaced. Accordingly, as compared to a case where the discharge lamp itself is processed to include the light diffusing structure thereon, the approach of providing the light diffusing structure on the protective tube is advantageous in that the light diffusing structure can be used for a longer time and thus the overall cost can be reduced, although an area of the protective tube to be processed is relatively great and the great to-be-processed area results in a high initial cost.

It should be appreciated that the light diffusing structure of the present invention is not intended for complete light diffusion but intended for such an appropriate degree of light diffusion as to lessen an undesired luminance unevenness. Therefore, the while-colored thin film in the present invention is formed to have an appropriate small thickness to allow the diffused light to pass through. Appropriate degree of the minute irregularities and translucency of the translucent ceramics is chosen for similar purposes.

Note that the term "luminance" refers to a ratio of luminous intensity in a given direction of an infinitesimal element of a surface containing a point of interest, to the orthogonally projected area of the element on a plane perpendicular to the given direction (from "Lighting Handbook" published by the lighting academic society that is a Japanese corporate judicial person), which is commonly used in relation to visible light. In this invention, the term will be used with a similar meaning in relation to ultraviolet rays.

### Brief Description of the Drawings

Fig. 1 is a partly-sectional side view showing an embodiment of an ultraviolet-utilizing treating apparatus in accordance with the present invention;
Fig. 2 is a partly-sectional side view showing an example of a discharge lamp employed in the embodiment of Fig. 1;
Fig. 3 is a sectional view showing an example of an outer tube (protective tube) employed in the embodiment of Fig. 1;
Fig. 4 is a graph showing results of experiments conducted by the treating apparatus of the present invention and a conventionally-known treating apparatus; and
Fig. 5 is a partly-sectional side view showing an example of a conventional treating apparatus.

### Best Mode for Carrying Out the Invention

Now, a detailed description will be made about embodiments of the present invention, with reference to the accompanying drawings.

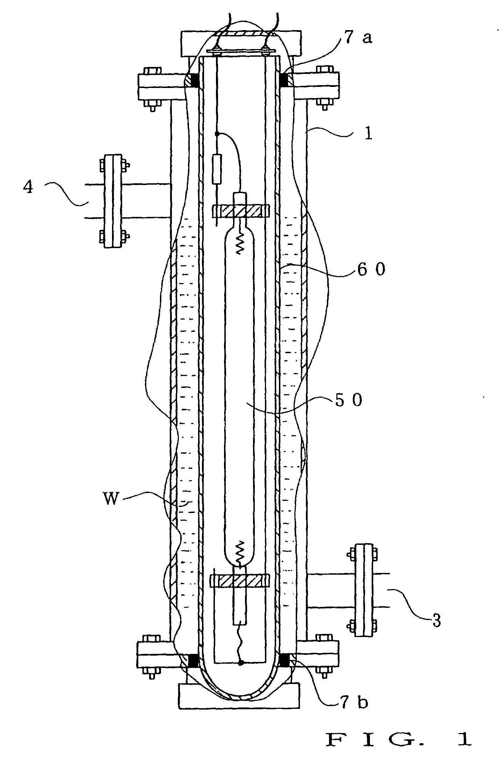

Fig. 1 shows an embodiment of a liquid treating apparatus in accordance with the present invention. The liquid treating apparatus has a vertically-extending cylindrical tank 1 made of stainless steel and a water inlet port 3 formed in its lower end portion, through which a substance (liquid) W to be treated is introduced into the treating apparatus. Ultraviolet ray discharge lamp 50 is inserted in and extends vertically along an outer tube (i.e., protective tube) 60 provided vertically within the cylindrical tank 1. The to-be-treated substance (liquid) W is subjected to sterilization, disinfection, etc. by ultraviolet rays emitted by the ultraviolet ray discharge lamp 50. The thus-treated substance (liquid) W is discharged through the water outlet port 4 disposed near the upper end of the cylindrical tank 1.

As an example, the outer tube (i.e., protective tube) 60 is a quartz-glass tube having a 22 mm inner diameter and 1.5 mm wall thickness, and it is retained at its upper and lower end, in a liquid tight manner, within the cylindrical tank 1 via rubber O rings 7a and 7b. The outer tube (i.e., protective tube) 60 has a top end opening upward to allow the discharge lamp 50 to be inserted or removed into or out of the outer tube 60. The outer tube 60 has a closed bottom end of a generally semicircular sectional shape.

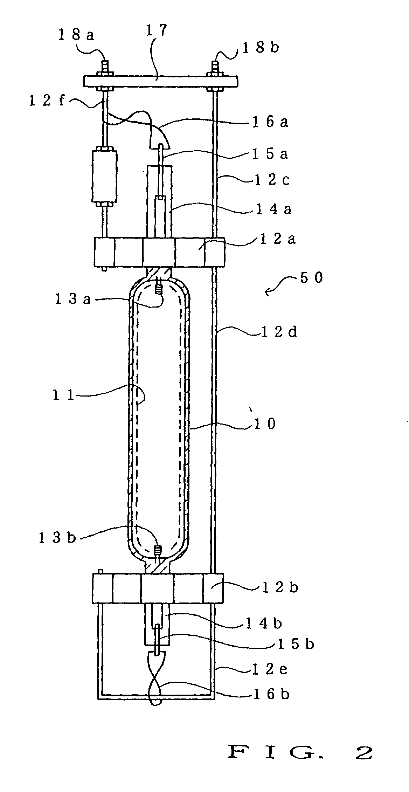

Fig. 2 shows an example of the ultraviolet ray discharge lamp 50 employed in the instant embodiment of the present invention. In the instant embodiment, the ultraviolet ray discharge lamp 50 is characterized in that a generally white-colored thin film 11 is provided in a light emitting tube 10. In the other respects, the ultraviolet ray discharge lamp 50 may be constructed similarly to the conventionally-known ultraviolet ray discharge lamps, as set forth below. Namely, as an example, the light emitting tube 10 is held in place by means of metal retaining members 12a, 12b, 12c, 12d, 12e and 12f, and a pair of electrodes 13a and 13b are provided within the light emitting tube 10 at opposite ends of the tube 10. The electrode 13a is electrically connected, via a molybdenum film 14a, wells 15a, external lead 16a and metal retaining member 12f, to a terminal 18a on an insulating plate 17, and the metal retaining members 12a and 12f are electrically insulated from each other by an insulator 19. The other electrode 13b is electrically connected, via a molybdenum film 14b, wells 15b, external lead 16b and metal retaining members 12e, 12d, 12c, to a terminal 18b on the insulating plate 17. As an example, the light emitting tube 10 is a quartz-glass tube having a 12 mm inner diameter and 1 mm wall thickness, and appropriate amounts of mercury and argon gas are sealed in the light emitting tube 10.

As an example, the generally white-colored thin film 11 is formed of minute powders of an aluminum oxide. The generally white-colored thin film 11 can be formed by applying a solution having the minute powders of aluminum oxide and binding agent suspended therein with butyl acetate and then, after drying, heating the minute powders in an oxidation atmosphere. Once the ultraviolet ray discharge lamp 50 is turned on or illuminated via a predetermined ballast (not shown), it can become a medium-pressure mercury vapor discharge lamp having a 700 V tube voltage, 1.7 A tube current and 1,000 W tube power. During the illumination, the ultraviolet ray discharge lamp 50 excites mercury atoms to produce ultraviolet rays, and the thus-produced ultraviolet rays are irradiated through the outer tube 60 to the to-be-treated substance. While a portion of the ultraviolet rays produced by arc discharge directly passes through the quartz-glass wall of the light emitting tube, the remaining portion of the ultraviolet rays is first diffused by the thin film 11 of aluminum oxide formed on the quartz-glass surface and then passes through the quartz-glass wall. As a consequence, ultraviolet rays with an lessened luminance non-uniformity or unevenness can be irradiated to the to-be-treated substance as uniformly as possible, which achieves enhanced purifying effects.

The generally white-colored thin film 11 formed on the inner surface of the discharge lamp 50 may be of any other suitable material than the above-mentioned aluminum oxide. For example, the thin film 11 may be formed using minute powers which have, as its main constituent, minute powers of at least one metal oxide selected from among a group consisting of a silicon oxide, calcium oxide, magnesium oxide, yttrium oxide and zirconium oxide.

If the generally white-colored thin film 11 for diffusing ultraviolet rays has a relatively great thickness, it can provide a near-complete diffusing surface and improve the irradiation uniformity of the ultraviolet rays; however, the thick generally white-colored thin film 11 is not preferable in that it results in a reduced light transmission rate. Therefore, in the present invention, it is preferable that the generally white-colored thin film 11 be formed into such an appropriate small thickness as to permit a lessened luminance non-uniformity. Experiments have confirmed that the generally white-colored thin film 11 of such an appropriate small thickness can afford sufficiently great benefits.

According to the present invention, the light diffusing structure provided on the discharge lamp 50 is not necessary limited to the formation of the white-colored thin film 11 as set forth above. For example, the light diffusing structure may be provided by forming minute projections and depressions or irregularities on the inner or outer glass surface of the light emitting tube 10 itself (i.e., the glass of the light emitting tube 10 may be formed as a frosted glass). Alternatively, the light emitting tube 10 may itself be formed of a translucent (diffusion-permeable) material, such as a polycrystal alumina ceramic material, to thereby provide the light diffusing structure. In these cases too, the light diffusing structure is formed in such a manner as to permit a lessened luminance unevenness and allow diffused ultraviolet rays to pass through the light emitting tube 10.

Of course, in the case where the light emitting tube 10 of the discharge lamp 50 is provided with the light diffusing structure as described above, the outer tube (i.e., protective tube) 60 may be constructed in the same manner as the conventional protective tube (e.g., protective tube 6 of Fig. 5). Further, where the to-be-treated substance is a gaseous or solid substance, the outer tube (protective tube) 60 may be dispensed with.

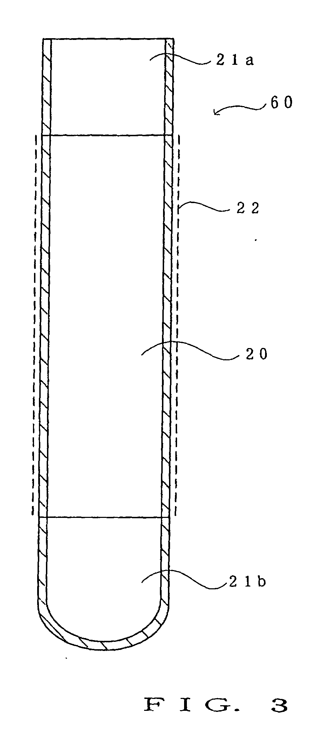

Fig. 3 is a view showing another embodiment of the present invention, where the light diffusing structure is provided on the outer tube (protective tube) 60. In this embodiment, the outer tube 60 has a portion 20 corresponding to a light emitting portion of a discharge lamp (not shown) inserted in the outer tube 60, and portions 21a and 21b corresponding to held or retained portions of the discharge lamp. The portion 20 corresponding to the light emitting portion of the discharge lamp has minute projections and depressions on its outer surface 22, i.e. the outer surface 22 is formed as a frosted glass surface, so that diffusion of ultraviolet rays is effected via the outer surface 22 of the portion 20. Such a minutely-rugged outer surface 22 may be formed using a mechanical grinding method called "dry honing". However, the present invention is not limited to such a mechanical grinding method, and the minutely-rugged outer surface 22 may also be formed by dipping the outer surface in a hydrofluoric acid solution to thereby chemically etch the surface. In another alternative, the minutely-rugged outer surface 22 may be formed on the inner surface of the outer tube (protective tube) 60. Whereas a generally white-colored thin film similar to the thin film 11, rather than the minutely-rugged outer surface 22, may be formed on the inner surface of the outer tube (protective tube) 60, this approach is not so advisable because there is a possibility of a certain object abutting against or hitting even such an inner surface of the outer tube (protective tube) 60. Further, where the light diffusing structure is provided on the outer tube (protective tube) 60 as in the example of Fig. 3, there is no need to provide the light diffusing structure on the discharge lamp 50, and thus a conventional discharge lamp (similar to the discharge lamp 5 of Fig. 5) may be employed.

In the case where the light diffusing structure is provided on the outer tube (protective tube) 60 as illustratively shown in Fig. 3, a portion of the ultraviolet rays produced by the discharge lamp 50 directly passes through the quartz-glass wall of the outer tube (protective tube) 60, the remaining portion of the ultraviolet rays is diffused by the minutely-rugged surface 22 formed on the quartz-glass surface 22 and then passes through the quartz-glass wall, in a similar manner to the above-described. As a consequence, ultraviolet rays with a lessened luminance non-uniformity can be irradiated to the to-be-treated substance as uniformly as possible, which achieves enhanced purifying effects.

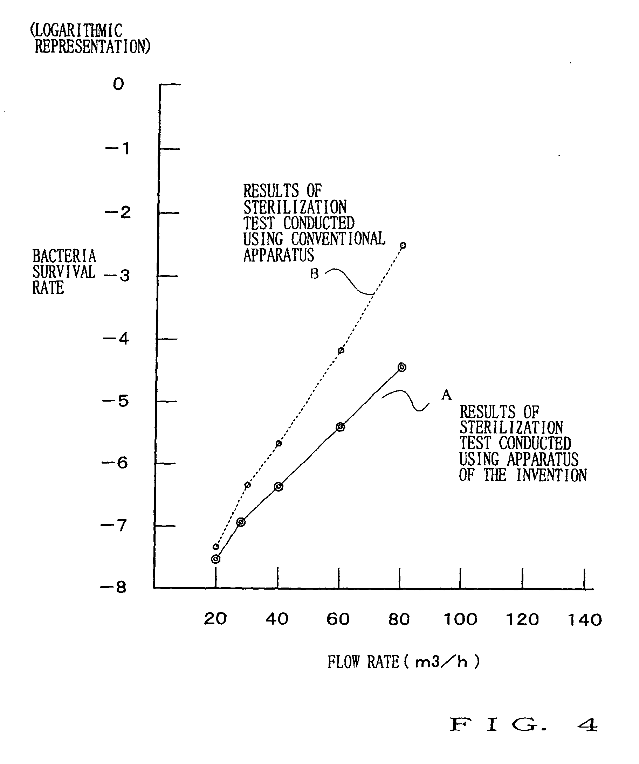

This and following paragraphs explain results of experiments conducted to ascertain the sterilizing performance of the embodiment of the present invention. Fig. 4 show results of sterilization tests using enterococcus, in which "A" represents results of the sterilization test which was conducted in accordance with the embodiment of the present invention while "B" represents results of the sterilization test which was conducted in accordance with the conventional technique. In each of the tests, a discharge lamp used as an ultraviolet ray light source was a medium-pressure mercury vapor discharge lamp having an effective light emission length of 35 cm and an input power of 1,000 watts. Specifically, the test B used a conventional discharge lamp, while the test A used the discharge lamp 50 where a generally white-colored thin film 11 is provided on the inner surface of the light emitting tube 10, as in the embodiment of Fig. 2, to lessen luminance non-uniformity. In Fig. 4, the horizontal axis represents a flow rate of a to-be-treated liquid, while the vertical axis represents a bacteria survival rate. More specifically, water containing 1.1 × 10⁵ live bacteria per milliliter was used as original to-be-treated water, and measurement was made of the number of the live bacteria at each flow rate, and the thus-obtained measurements are plotted in the figure as the bacteria survival rate. As shown in the figure, when the flow rate was low, the two tests showed very low bacteria survival rates that did not differ from each other so much. But, as the flow rate increased, the test A showed much higher sterilizing performance than the test B. Although not particularly shown, another experiment, conducted in relation to an apparatus where a light diffusion structure for lessening luminance non-uniformity was disposed on the outer tube 60 as in the embodiment of Fig. 3, also confirmed that the apparatus can present sterilizing performance generally as high as that confirmed by the test A.

Whereas the embodiments and experiment results shown in the drawings have been described above in relation to a treating apparatus including only one discharge lamp, it has been confirmed through an experiment that multi-lamp-type sterilizing apparatus of a greater capacity can achieve enhancement of the sterilizing performance substantially proportional to the increased number of the discharge lamps by applying the basic principles of the present invention to each of the lamps.

It should be appreciated that the construction of the inventive treating apparatus utilizing ultraviolet rays may be of any other desired type than the sealed type where a to-be-treated liquid is introduced into a cylinder-shaped tank like the tank 1 of Fig. 1 and subjected to irradiation of ultraviolet rays. For example, the present invention can be effectively applied to a type of treating apparatus where an ultraviolet ray light source is directly immersed in an open channel or embedded sewer or drainpipe to perform sterilizing/disinfecting processing on dirty water. In addition, the present invention is applicable to any organic-substance or hazardous-substance decomposing apparatus, other than sterilizing/disinfecting apparatus, as along as the apparatus uses ultraviolet ray energy to treat a to-be-treated substance, whether the to-be-treated substance is in liquid form or in gaseous form.

Further, the embodiments have been described above in relation to the case where the ultraviolet ray light source is a medium-pressure mercury vapor discharge lamp capable of power input in the order of 1,000 watts. However, the present invention is not so limited, and the present invention is applicable to cases where is employed any desired type of ultraviolet ray light source, such as a medium-pressure discharge lamp of higher power input, high-pressure mercury vapor discharge lamp or discharge lamp having no electrode.

As having been described so far, the present invention is characterized by inclusion of the light diffusing structure for diffusing ultraviolet rays, emitted from the ultraviolet ray light source, to irradiate the diffused ultraviolet rays to a to-be-treated substance. With such an arrangement, the ultraviolet rays can be irradiated to the to-be-treated substance in a diffused manner. As a result, the present invention can advantageously lessen a non-uniformity or unevenness in the luminance of the ultraviolet rays irradiated to the to-be-treated substance, so that as great a part of the to-be-treated substance as possible can be irradiated with the ultraviolet rays as uniformly as possible, and the invention can thereby greatly enhance the processing capabilities for sterilization or the like of the to-be-treated substance. Particularly, if a high-density light source is employed, the present invention allows a much greater part of the to-be-treated substance to be subjected to increased amounts of ultraviolet ray irradiation, and can thereby provide enhanced processing capabilities commensurate with the increased ultraviolet ray irradiation amounts.

## Claims

1. A treating apparatus for purifying a to-be-treated substance with ultraviolet ray energy emitted by an ultraviolet ray light source positioned near the to-be-treated substance,
**characterized by** including a light diffusing structure that diffuses ultraviolet rays, emitted by said ultraviolet ray light source, to irradiate the to-be-treated substance with the diffused ultraviolet rays, so as to lessen unevenness in luminance.

2. A treating apparatus as claimed in claim 1 wherein said light diffusing structure includes a generally while-colored thin film formed on an inner surface of a glass tube constituting a light emitting tube of said ultraviolet ray light source.

3. A treating apparatus as claimed in claim 2 wherein the generally while-colored thin film formed on the inner surface of the glass tube has, as a main constituent thereof, at least one metal oxide selected from among a group consisting of an aluminum oxide, silicon oxide, calcium oxide, magnesium oxide, yttrium oxide, zirconium oxide and hafnium oxide.

4. A treating apparatus as claimed in claim 1 wherein said light diffusing structure is provided by forming minute irregularities on an inner surface of a glass tube constituting a light emitting tube of said ultraviolet ray light source.

5. A treating apparatus as claimed in claim 1 wherein said light diffusing structure is provided by forming a light emitting tube of said ultraviolet ray light source of a translucent ceramic material.

6. A treating apparatus as claimed in claim 1 wherein said light diffusing structure is provided by accommodating said ultraviolet ray light source in a light-transmissive protective tube and forming minute irregularities on an inner or outer surface of the protective tube.

7. A treating apparatus as claimed in claim 1 wherein said light diffusing structure is provided by accommodating said ultraviolet ray light source in a light-transmissive protective tube and forming the protective tube of a translucent ceramic material.
